# EUROPÄISCHE PATENTSCHRIFT

(11) **EP 1 212 993 B1**
(45) Veröffentlichungstag und Bekanntmachung des Hinweises auf die Patenterteilung: **16.06.2010**
(21) Anmeldenummer: 01127280.4
(22) Anmeldetag: 16.11.2001
(51) Int. Cl.: A61F 2/46

(54) **Mischvorrichtung und Abgabegerät für eine fliessfähige Substanz**
Mixing device and dispensing device for a free-flowing substance
Dispositif de mélange et dispositif de transfer d'une matière à écoulement libre

(30) Priorität: 21.11.2000 DE 10057616
(43) Veröffentlichungstag der Anmeldung: 12.06.2002
(73) Patentinhaber: Stryker Trauma GmbH, 24232 Schönkirchen (DE)
(72) Erfinder: Speitling, Andreas, Dr., 24149 Kiel (DE); von Oldenburg, Geert, 24105 kiel (DE)
(74) Vertreter: Kopf, Korbinian Paul

(56) Entgegenhaltungen:
- FR-A- 2 572 677
- US-A- 5 167 448
- US-A- 5 395 167
- US-A- 6 099 160

## Beschreibung

Die Erfindung bezieht sich auf eine Mischvorrichtung zum Mischen einer aus zwei Komponenten bestehenden fließfähigen Substanz, insbesondere Knochenzement, nach dem Patentanspruch 1.

Knochenzement setzt sich bekanntlich aus einer pulverförmigen und einer flüssigen Komponente zusammen, die nach dem Vermischen rasch reagieren. Die fertige Mischung muss daher schnellstmöglich verarbeitet werden. Die pulverförmige Komponente wird üblicherweise steril in einer Blisterpackung oder in einer Flasche abgefüllt, beispielsweise mit einem Inhalt von 2 bis 25 g. Die Flüssigkeit, z.B. eine molare Natriumphosphatlösung, destilliertes Wasser oder eine Natriumchloridlösung liegt üblicherweise in einer sterilen Kunststoff-Wegwerfspritze vor mit einem Volumen von 10 cc. Üblicherweise werden die beiden Komponenten in einem Gefäß vermischt und aus diesem Gefäß heraus verarbeitet, beispielsweise mit Hilfe einer Spritze oder dergleichen. Das Vermischen muss intensiv sein, andererseits darf die Mischzeit nicht zu lange dauern.

Eine Mischvorrichtung ist beispielsweise bekannt aus US 5,167,448, bei der eine Mischvorrichtung vorgesehen ist, mit einem Mischbehälter auf einem Rotor für eine Drehung relativ zu dem Rotor um eine Achse, die exzentrisch im Hinblick auf die Rotorachse ausgebildet ist. Dabei erfolgt eine Überlagerung von zwei Drehbewegungen, die bei einer entsprechenden Ausgestaltung eines Übersetzungsverhältnisses zu einer Parallelbewegung des Behälters entlang einer Kreisbahn führt.

Eine analoge Anordnung ist der US 6,099,160 zu entnehmen, bei der eine Überlagerung von zwei Rotationsbewegungen vorgenommen wird.

Der Erfindung liegt die Aufgabe zugrunde, ein Verfahren zum Mischen und zum Applizieren einer fließfähigen Substanz anzugeben, insbesondere Knochenzement, das besonders einfach und wirksam durchgeführt werden kann.

Diese Aufgabe wird erfindungsgemäß durch folgende Schritte gelöst:

Die pulverförmige Komponente wird in eine Injektionsspritze eingefüllt, die normalerweise einen Kolben an einem Ende und ein Abgabeende hat, auf das normalerweise eine Kanüle aufgesteckt wird. Die Kanüle ist jedoch entfernt und statt dessen wird ein geeigneter Verschluss aufgebracht, beispielsweise eine Kappe. Anschließend wird die zweite flüssige Komponente in die Injektionsspritze eingefüllt, vorzugsweise über eine mit der flüssigen Komponente gefüllte zweite Spritze über eine daran angebrachte Kanüle. Danach wird die erste Spritze mit dem Spritzenkolben verschlossen unter Belassung ausreichender Luft im Spritzenzylinder. Es ist auch denkbar, daß beide Komponenten sich bereits in einer Spritze befinden, jedoch durch eine Trennwand voneinander getrennt sind. Zum Mischen wird die Trennwand entfernt oder durchlässig gemacht.

Danach wird die Spritze intensiv geschüttelt zwecks Vermischung der Komponenten. Anschließend wird der Verschluss entfernt und eine Kanüle auf die erste Spritze aufgesetzt, wonach dann die fließfähige Substanz an einem gewünschten Ort, beispielsweise am versorgten Knochen ausgetragen wird.

Die Erfindung geht davon aus, dass die flüssige Komponente beispielsweise in einer Wegwerfspritze abgepackt ist, mithin relativ einfach in eine Spritze eingefüllt werden kann, welche die pulverförmige Komponente enthält. Diese Spritze dient dann als Mischbehälter. Sie ist an den Enden verschlossen. Daher kann sie heftig bewegt werden, beispielsweise auch in einer maschinellen Mischvorrichtung, damit innerhalb kürzester Zeit eine intensive Vermischung durchgeführt werden kann.

Die Erfindung bezieht sich auch auf eine Mischvorrichtung, mit der eine fließfähige Substanz, die aus zwei Komponenten besteht, intensiv in kürzester Zeit vermischt werden kann, beispielsweise zur Bildung von Knochenzement. Eine derartige Mischvorrichtung sieht eine Injektionsspritze mit einem Kolben und einem Verschluss am vorderen Ende als Mischbehälter vor für die eingefüllten Komponenten. Die pulverförmige Komponente kann vergleichbar der flüssigen Komponente ebenfalls in einer Spritze steril abgepackt sein. Es ist jedoch auch möglich, wie oben beschrieben, die pulverförmige Komponente nach dem Entfernen des Spritzenkolbens in den Zylinder einzufüllen, wonach dann auch die flüssige Komponente eingefüllt wird. Anschließend wird der Kolben in den Zylinder eingeführt unter Belassung eines ausreichenden Freiraums zu Mischzwecken. Die Mischvorrichtung weist ferner einen Halter auf zur lösbaren Halterung der Spritze sowie eine Mischkinematik, die dem Halter eine Bewegung erteilt, die sich aus der Überlagerung einer rotatorischen und einer linearen translatorischen Bewegung ergibt. Die Kinematik wird durch eine Eingangswelle in Gang gesetzt, die entweder von Hand angetrieben ist oder von der Spindel einer Drehantriebsmaschine, z.B. eine pneumatische oder elektrisch angetriebene chirurgische Maschine oder einem separaten Elektromotor. Vorzugsweise ist dann zwischen der Spindel und der Eingangswelle der Mischkinematik ein Eingangsgetriebe angeordnet. Das Eingangsgetriebe setzt die Drehzahl angemessen herauf oder herab, bevor sie auf die Mischkinematik gegeben wird. Die Welle des Eingangsgetriebes weist eine entsprechende Aufnahme für die Abtriebsspindel der Drehantriebsmaschine, die einen speziellen in die Aufnahme einsteckbaren Aufsatz aufweisen kann.

Der Halter für die Injektionsspritze der erfindungsgemäßen Vorrichtung ist nach einer weiteren Ausgestaltung der Erfindung als Pleuelstange eines Pleueltriebs ausgebildet. Zu diesem Zweck kann das Ende des Halters exzentrisch an einem Rotor angelenkt sein, der mit der Ausgangswelle des Eingangsgetriebes gekoppelt ist, während das andere Ende linear beweglich gelagert ist.

Nach dem Mischen und Entfernen der Spritze kann diese über ein Abgabegerät betätigt werden. Erfindungsgemäß weist ein derartiges Abgabegerät eine Aufnahme für eine Injektionsspritze mit Kolben derart auf, dass das Abgabeende der Spritze von Hand zugänglich ist für die Abnahme eines Verschlusses auf dem Abgabeende und das Aufsetzen einer Kanüle. Das Abgabegerät weist eine Vorschubstange auf, die mit dem Kolbenende der Spritze zusammenwirkt und von einem Handhebel betätigt wird, dessen Bewegung über ein Getriebe auf die Vorschubstange übertragen wird. Derartige Spritzpistolen sind für andere Zwecke bereits bekannt geworden, beispielsweise zum Auspressen von Dichtungsmaterial aus einer Kartusche, die in eine Aufnahme der Spritzpistole eingelegt wird.

Eine sichere Aufnahme der Spritze in dem Halter besteht erfindungsgemäß darin, dass dieser einen Kanal aufweist, in welchen die Spritze eingelegt wird. Außerdem hat der Kanal in Abständen Quereinschnitte, in welche der Flansch des Spritzenzylinders eingelegt werden kann. Auf diese Weise können verschieden lange Spritzen in vollem Halter sicher aufgenommen werden. Zur Festlegung der Spritze am Halter ist vorzugsweise ein Steg vorgesehen, der die Spritze überspannt und mit Hilfe von Schrauben oder dergleichen am Halter festlegbar ist, um den Spritzenzylinder klemmend festzulegen.

Die Erfindung soll nachfolgend anhand eines in Zeichnungen dargestellten Ausführungsbeispiels näher erläutert werden.
- Fig. 1: zeigt eine Mischvorrichtung nach der Erfindung in perspektivischer Darstellung.
- Fig. 2: zeigt eine Draufsicht auf die Mischvorrichtung mit aufgenommener Injektionsspritze.
- Fig. 3: zeigt schematisch die Kinematik zum Antrieb eines Halters der Mischvorrichtung nach den Figuren 1 und 2.

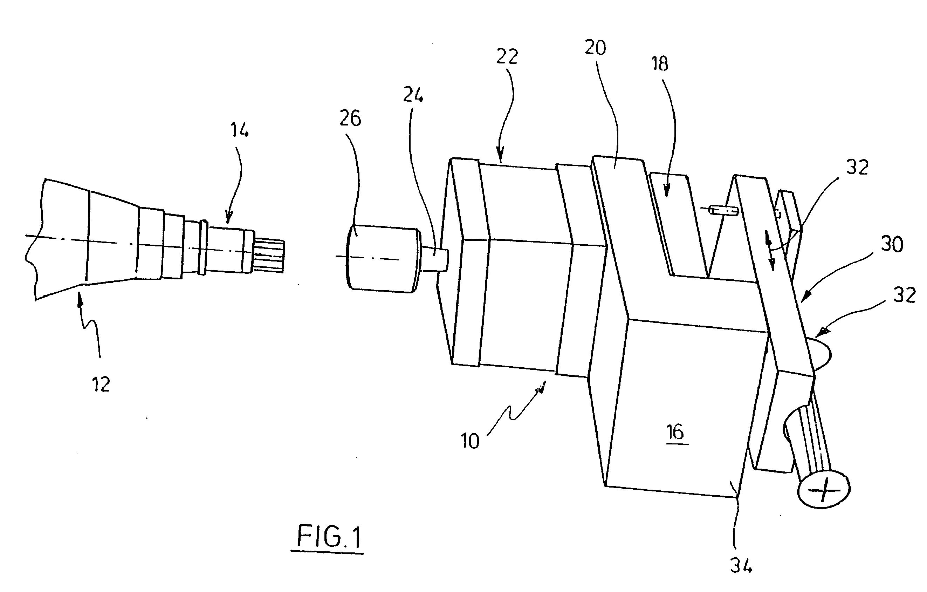

In Fig. 1 ist eine Mischvorrichtung 10 schematisch dargestellt sowie eine Drehantriebsmaschine 12 angedeutet mit einer Antriebsspindel 14.

Die Mischvorrichtung 10 weist ein allgemein L-förmiges Basisteil 16 auf, an dessen einem Schenkel innen eine Scheibe 18 drehbar gelagert ist. Auf der gegenüberliegenden Seite des Schenkels 20 befindet sich ein Untersetzungsgetriebe 22, das mit der Lagerwelle (nicht gezeigt) der Scheibe 18 über seine Ausgangswelle (nicht gezeigt) in Eingriff ist. Eine Eingangswelle 24 des Eingangsgetriebes 22 weist eine Aufnahme 26 auf für die drehfeste Aufnahme der Antriebsspindel 14 der Drehantriebsmaschine 12. Zu Kopplungszwecken wird die Aufnahmespindel eingesteckt.

Ein länglicher Halter 30 für eine Injektionsspritze 32 ist in seiner Längsrichtung gemäß Doppelpfeil 32 linear beweglich am anderen Schenkel 34 des Basisteils 16 gelagert. Ein nicht gezeigter Zapfen der Scheibe 18 ist in Drehverbindung mit der Unterseite des Halters 30. Der nicht gezeigte Zapfen ist exzentrisch auf der Scheibe 18 angebracht. Eine Drehung der Scheibe 18 führt daher zu einer überlagerten Bewegung aus der Rotation der Scheibe 18 und der erzwungenen translatorischen Bewegung des Halters 30.

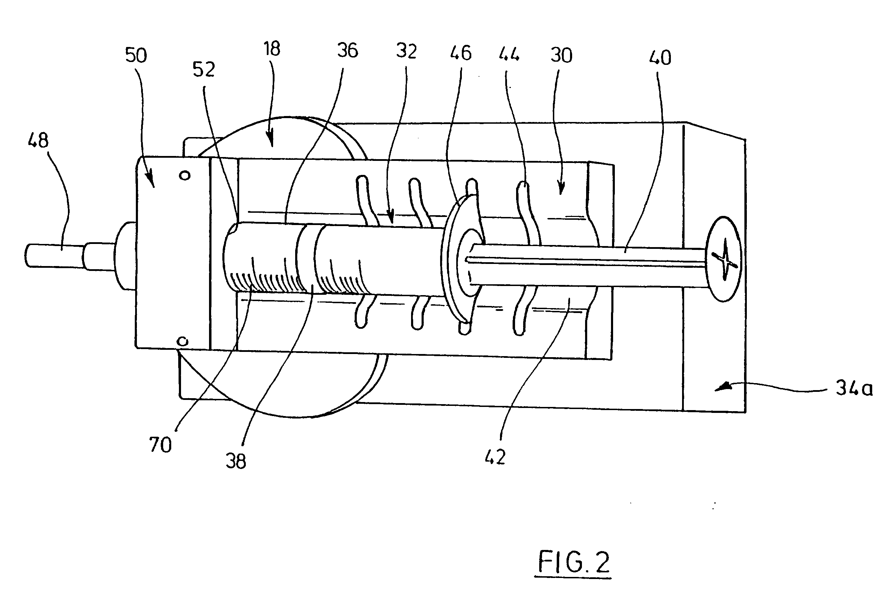

In Fig. 2 ist der Halter 30 deutlicher dargestellt. Das Bauteil 34a, auf dem der Halter 30 linear beweglich gelagert ist, unterscheidet sich etwas von dem nach Fig. 1. Für die Wirkungsweise hat dies jedoch keine Bedeutung.

Man erkennt außerdem die Scheibe 18, die exzentrisch an der Unterseite des Halters 30 angelenkt ist. Die Spritze 32 ist eine herkömmliche Injektionsspritze mit einem Spritzenzylinder 36, einem Kolben 38 und einer Kolbenstange 40. Der Halter 30 weist an der Oberseite einen im Querschnitt annähernd halbkreisförmigen Kanal 42 auf, in den der Spritzenzylinder 36 eingelegt wird. Quer zum Kanal 32 sind in Abständen Einschnitte 44 geformt, die einen Flansch 46 am Ende des Zylinders 36 aufnehmen. Das Abgabeende der Spritze 32 ist durch eine Verschlusskappe 48 verschlossen. Ein stegartiges Bauteil 50 weist eine kanalförmige Ausnehmung 52 auf, die ein Teil des Spritzenzylinders 36 aufnimmt, wenn es, wie in Fig. 2 gezeigt ist, über die Spritze 32 gelegt wird, um diese festzuklemmen. Die Befestigung des Stegs 50 am Halter 30 erfolgt mit Hilfe von Schrauben oder ähnlichen lösbaren Befestigungsmitteln.

Eine Spritze wie die Spritze 32 wird zunächst außerhalb des Halters 30 und bei entferntem Kolben 38 mit den beiden Komponenten, die vermischt werden sollen, beispielsweise eine pulverförmige Knochenzementkomponente und eine flüssige Komponente, nacheinander befüllt. Anschließend wird der Kolben eingeschoben unter Belassung ausreichenden Zwischenraums. Danach wird die durch die Kappe verschlossene Spritze 48 in den Halter 30 eingelegt und befestigt. Danach kann durch Aufbringen eines Drehmoments über das Eingangsgetriebe 22 die Mischbewegung in Gang gesetzt werden.

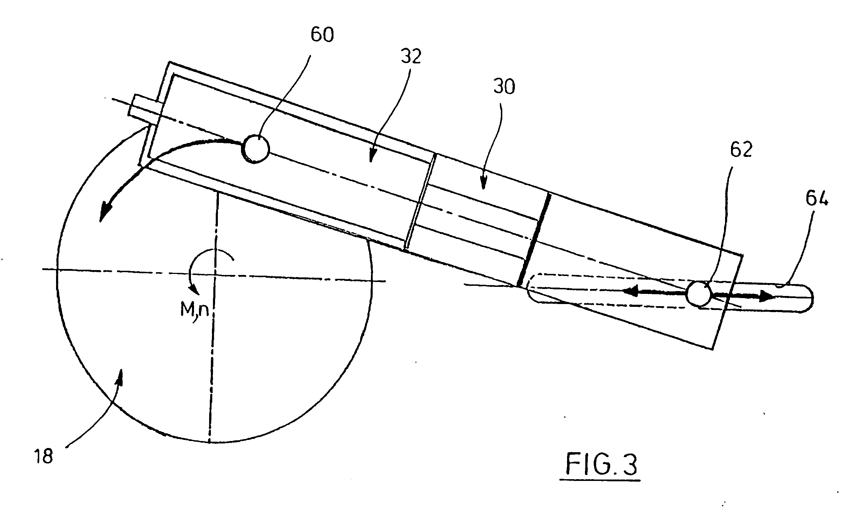

Für die Mischbewegung dient eine Kinematik, wie sie in Fig. 3 schematisch dargestellt ist. Die Scheibe 18 ist über einen Zapfen 60 mit dem Halter 30 gekoppelt, der einen Zapfen 62 aufweist, der in einem länglichen Schlitz 64 des Bauteils 34a linear geführt ist. Bei der Rotation der Scheibe 18 führt die Spritze 32 mit dem Halter 30 eine Schwenkbewegung um den Zapfen 62 aus mit einer Amplitude, welche durch den Radius der Position des Zapfens 60 bestimmt ist. Die Amplitude ist ferner abhängig von der Lage der Spritze im Halter bzw. der Lage des Halters im Zapfen 60 Gleichzeitig wird bei einer Umdrehung der Scheibe 18 der Zapfen 60 im Längsschlitz 64 hin und her bewegt, wobei der Hub ebenfalls vom Radius der Position des Zapfens 60 abhängig ist.

Nach dem Mischvorgang, der z.B. 30 Sekunden dauert, wird die Spritze 32 vom Halter 30 entfernt und in ein Abgabegerät eingegeben, das die Spritze mit Kolben aufzunehmen in der Lage ist. Dabei ist das vordere Ende der Spritze 32 von Hand zugänglich, damit die Kappe 48 entfernt und eine Kanüle aufgesetzt werden kann. Das Abgabegerät (nicht gezeigt) weist eine Vorschubstange auf, die mit dem Kolben bzw. der Kolbenstange 40 der Spritze 32 zusammenwirkt. Die Vorschubstange wird ihrerseits von einem Handhebel betätigt über ein geeignetes Getriebe. Das Getriebe kann so gewählt werden, dass mit einem einzigen Hub des Handhebels der gesamte Inhalt der Spritze 32 ausgepresst werden kann. Es kann jedoch auch so ausgelegt werden, dass mehrere Hübe des Handhebels erforderlich sind. Da die Spritze 32 eine Markierung 70 aufweist, kann beobachtet werden, wieviel Inhalt jeweils mit einem Hub des Handhebels abgegeben wird.

Das Zusammenbringen der Komponenten und das Haltern der Spritze in der Mischvorrichtung soll nach Möglichkeit weniger als 1 Minute dauern. Bei entsprechender Drehgeschwindigkeit der Scheibe 18 kann eine Vermischung innerhalb von 30 Sekunden durchgeführt werden. Das Herausnehmen der Spritze, das Einbringen in ein Abgabegerät und das Verarbeiten soll nach Möglichkeit nicht länger als 5 Minuten dauern.

Die in den Fig. 1 und 2 gezeigte Vorrichtung kann zwecks besserer Handhabung auf einem Untergrund z.B. einer Tischplatte oder einem speziellen Gestell lösbar befestigt werden.

## Patentansprüche

1. Mischvorrichtung für eine aus einer pulverförmigen ersten Komponente und einer flüssigen zweiten Komponente bestehende fließfähige Substanz, insbesondere Knochenzement, mit den folgenden Merkmalen:
- eine Injektionsspritze (32) mit einem Kolben (38) und einem entfernbaren Verschluss (48) am Abgabeende als Mischbehälter für die eingefüllten Komponenten,
- ein Halter (30) für die lösbare Halterung der Spritze (32), und
- eine Mischkinematik, die dem Halter (30) eine Bewegung erteilt, die sich aus der Überlagerung einer rotatorischen und einer translatorischen Bewegung ergibt, **dadurch gekennzeichnet, daß** die translatorische Bewegung linear ist.

2. Vorrichtung nach Anspruch 1, **dadurch gekennzeichnet, dass** ein Eingangsgetriebe (22) vor der Mischkinematik angeordnet ist und die Eingangswelle (24) für das Eingangsgetriebe (22) mit einer Aufnahme (26) für die Spindel (14) einer Drehantriebsmaschine (12) versehen ist.

3. Vorrichtung nach Anspruch 1 oder 2, **dadurch gekennzeichnet, dass** der Halter (30) als Pleuelstange eines Pleueltriebs wirkt.

4. Vorrichtung nach Anspruch 3, **dadurch gekennzeichnet, dass** ein Endbereich des Halters (30) exzentrisch an einen Rotor (18) angelenkt ist, der mit der Ausgangswelle des Eingangsgetriebes (22) gekoppelt ist, während das andere Ende linear beweglich gelagert ist.

5. Vorrichtung nach einem der Ansprüche 1 bis 4, **dadurch gekennzeichnet, dass** der Verschluss (48) der Spritze (32) eine Kappe ist.

6. Vorrichtung nach einem der Ansprüche 1 bis 3, **dadurch gekennzeichnet, dass** der Halter (30) einen Aufnahmekanal (42) aufweist, der in Abständen Quereinschnitte (44) aufweist für den Flansch (46) des Spritzenzylinders (36).

7. Verfahren zum Mischen einer fließfähigen Substanz, die aus einer pulverförmigen ersten Komponente und einer flüssigen zweiten Komponente besteht, insbesondere Knochenzement, mit den folgenden Verfahrensschritten:
- Einfüllen der ersten Komponente in eine Injektionsspritze nach dem Entfernen des Spritzenkolbens und dem Aufsetzen eines Verschlusses auf das Abgabeende der Spritze,
- Zusammenbringen der flüssigen zweiten Komponente in der Spritze mit der ersten Komponente, vorzugsweise Einfüllen der zweiten Komponente aus einer zweiten, mit der flüssigen Komponente gefüllten zweiten Spritze über eine Kanüle der zweiten Spritze,
- Verschließen der ersten Spritze am anderen Ende mit dem Spritzenkolben unter Belassung ausreichender Luft im Spritzenzylinder, und
- Mischen der Komponenten durch Schütteln der ersten Spritze unter Verwendung der Mischvorrichtung nach einem der Ansprüche 1 bis 6.

8. Verfahren nach Anspruch 7, **dadurch gekennzeichnet, dass** die erste Spritze in eine Spritzpistole eingelegt wird, bei der mit Hilfe eines Handhebels eine Vorschubstange betätigt wird, die ihrerseits auf den Kolben der ersten Spritze wirkt.

## Claims

1. A mixing device for a flowable substance, in particular bone cement, the flowable substance consisting of a powdered first component and a liquid second component, the mixing device having the following features:
- an injection syringe (32) having a plunger (38) and a removable closure (48) at the delivery end as a mixing container for the components poured into it,
- a holder (30) for the releasable mounting of the syringe (32), and
- mixing kinematics imparting to the holder (30) a movement, which results from superimposing a rotational movement and a translational movement,
**characterized in that**
the translational movement is linear.

2. The device according to claim 1,
**characterized in that**
an inlet gear (22) is arranged in front of the mixing kinematics, and the input shaft (24) for the inlet gear (22) is provided with a receptacle (26) for the spindle (14) of a rotary drive machine (12).

3. The device according to claim 1 or 2,
**characterized in that**
the holder (30) acts as a connecting rod of a connecting rod drive.

4. The device according to claim 3,
**characterized in that**
an end area of the holder (30) is eccentrically hinge-connected to a rotor (18), which is coupled to the output shaft of the inlet gear (22), while the other end is mounted to be linearly movable.

5. The device according to any one of claims 1 to 4, **characterized in that** the closure (48) of the syringe (32) is a cap.

6. The device according to any one of claims 1 to 3, **characterized in that**
the holder (30) has a receiving channel (42), having transverse cuts (44) at intervals for the flange (46) of the syringe barrel (36).

7. A method for mixing a flowable substance, for a flowable substance, in particular bone cement, the flowable substance consisting of a powdered first component and a liquid second component, the method having the following features:
- filling the first component into an injection syringe after removing the syringe plunger and attaching a closure to the delivery end of the syringe,
- bringing together the liquid second component in the syringe with the first component, preferably pouring the second component from a second syringe filled with the liquid component through a cannula of the second syringe,
- sealing the first syringe at the other end with the syringe plunger, leaving sufficient air in the syringe barrel, and
- mixing the components by shaking the first syringe, using the mixing device according to any one of claims 1 to 6.

8. The method according to claim 7,
**characterized in that**
the first syringe is inserted into a syringe gun, with which a pusher rod is operated with the help of a hand lever, acting in turn on the plunger of the first syringe.

## Revendications

1. Dispositif de mélange pour une substance coulante constituée par un premier composant poudreux et un second composant liquide, notamment un ciment osseux, comprenant les caractéristiques suivantes :
- une seringue (32) comprenant un piston (38) et un élément de fermeture amovible (48) au niveau de l'extrémité de libération en tant que récipient de mélange pour les composants versés,
- un support (30) pour la fixation détachable de la seringue (32), et
- une cinématique de mélange, qui communique au support (30) un mouvement, qui est obtenu à partir de la superposition d'un mouvement rotatif et translationnel, **caractérisé en ce que** le mouvement translationnel est linéaire.

2. Dispositif selon la revendication 1, **caractérisé en ce qu'**un mécanisme d'entrée (22) est agencé avant la cinématique de mélange et l'arbre d'entrée (24) pour le mécanisme d'entrée (22) est équipé d'un logement (26) pour la broche (14) d'une machine d'entraînement en rotation (12).

3. Dispositif selon la revendication 1 ou 2, **caractérisé en ce que** le support (30) agit en tant que bielle d'un entraînement par bielle.

4. Dispositif selon la revendication 3, **caractérisé en ce qu'**une zone d'extrémité du support (30) est articulée de manière excentrique sur un rotor (18), qui est couplé à l'arbre de sortie du mécanisme d'entrée (22), tandis que l'autre extrémité est logée de manière à pouvoir effectuer des mouvements linéaires.

5. Dispositif selon l'une des revendications 1 à 4, **caractérisé en ce que** l'élément de fermeture (48) de la seringue (32) est un capuchon.

6. Dispositif selon l'une des revendications 1 à 3, **caractérisé en ce que** le support (30) présente un canal de logement (42), qui présente à intervalles des rainures transversales (44) pour le collet (46) du cylindre de seringue (36).

7. Procédé de mélange d'une substance coulante, qui est constituée par un premier composant poudreux et un second composant liquide, notamment un ciment osseux, comprenant les étapes de procédé suivantes :
- versement du premier composant dans une seringue après le retrait du piston de seringue et pose d'un élément de fermeture sur l'extrémité de libération de la seringue,
- regroupement du second composant liquide dans la seringue avec le premier composant, de préférence versement du second composant depuis une seconde seringue remplie du composant liquide via une canule de la seconde seringue,
- fermeture de la première seringue à l'autre extrémité avec le piston de seringue en laissant suffisamment d'air dans le cylindre de seringue, et
- mélange des composants par agitation de la première seringue en utilisant le dispositif de mélange selon l'une des revendications 1 à 6.

8. Procédé selon la revendication 7, **caractérisé en ce que** la première seringue est introduite dans un pistolet pulvérisateur, dans lequel à main un poussoir est actionné à l'aide d'un levier, lequel poussoir agit de son côté sur le piston de la première seringue.
